**Europäisches Patentamt**

**European Patent Office**

**Office européen des brevets**

(11) Veröffentlichungsnummer: **0 135 123**
**A2**

(12) # EUROPÄISCHE PATENTANMELDUNG

(21) Anmeldenummer: **84109533.4**

(22) Anmeldetag: **10.08.84**

(51) Int. Cl.⁴: **C 12 N 1/38**
**//C12P19/06**

(30) Priorität: **23.08.83 DE 3330328**

(43) Veröffentlichungstag der Anmeldung:
**27.03.85 Patentblatt 85/13**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Voelskow, Hartmut, Dr.**
**Akazienstrasse 22**
**D-6234 Hattersheim am Main(DE)**

(72) Erfinder: **Keller, Reinhold, Dr.**
**Wiesenweg 5**
**D-6232 Bad Soden am Taunus(DE)**

(72) Erfinder: **Schlingmann, Merten, Dr.**
**Schneidhainer Strasse 32a**
**D-6240 Königstein/Taunus(DE)**

(54) **Verfahren zur Verringerung der Viskosität von Fermentationsbrühen.**

(57) Die Viskosität von Fermentationsbrühen wird durch Zusatz eines ungiftigen, flüssigen hochhalogenierten Aliphaten stark verringert. Bei aeroben Fermentationen ergibt sich als zusätzlicher Vorteil der verbesserte Sauerstofftransport durch den hochhalogenierten Aliphaten. Auch beim Einsatz hydrophiler Lösemittel bei der Aufarbeitung des Fermentationsansatzes ergibt sich eine einfache Aufarbeitung, da der hochhalogenierte Aliphat mit diesen Lösemitteln nicht mischbar ist und somit durch einfaches Dekantieren von der hydrophilen Phase abgetrennt werden kann.

Croydon Printing Company Ltd

Verfahren zur Verringerung der Viskosität von Fermentationsbrühen.

Die Erfindung betrifft ein Verfahren zur Verringerung der
Viskosität von Fermentationsbrühen, bei dem die Fermentation in Gegenwart eines ungiftigen, unter den Fermentationsbedingungen flüssigen hochhalogenierten Aliphaten
durchgeführt wird.

Aus der US-Patentschrift 4 352 882 ist es bekannt, Xanthan
durch Fermentation herzustellen, wobei das wässerige
Kulturmedium in einem wasserunmischbaren Öl dispergiert
wird, das kein Lösemittel für das Produkt darstellt. Hierbei wird das Öl zu dem Zeitpunkt zugegeben, zu dem normalerweise die Reaktion abgebrochen wird, nämlich wenn
die Reaktionsmasse zu viskos wird und nicht länger gerührt
werden kann. Die Ölmengen betragen etwa 20 bis 80 %, vorzugsweise 40 bis 80 %, bezogen auf das Gewicht des wässerigen
Kulturmediums. Als Öle kommen alle wasserunmischbaren, in
Wasser dispergierbaren organischen Öle in Betracht, vorzugsweise höher - siedende flüssige Kohlenwasserstoffe oder
pflanzliche Öle; auch ein Silikonöl wurde eingesetzt.
Ferner wird erwähnt, daß auch bestimmte halogenierte Kohlenwasserstoffe brauchbar sein sollen.

Eigene Untersuchungen haben allerdings gezeigt, daß bei
diesem bekannten Verfahren Emulsionen entstehen, wodurch
die Viskosität nicht nur nicht verringert, sondern sogar
erhöht wird. Diese Gefahr besteht insbesondere beim Einsatz
von Emulgatoren, eine Ausführungsform, die bei dem bekannten
Verfahren als bevorzugt bezeichnet wird. Vor allem bei der
fermentativen Herstellung von extrazellulären Polysacchariden kann es so zu stabilen Emulsionen kommen, da
das Biopolymer die Emulsionen ebenfalls stabilisiert.

Demgegenüber führen hochhalogenierte Aliphaten, insbesondere
perhalogenierte und vor allem perfluorierte Verbindungen zu relativ großen Tröpfchen, so daß auch bei der Synthese von Exopolysacchariden keine stabilen Emulsionen gebildet werden. Es hat sich gezeigt, daß die Tröpfchengröße nicht wesentlich kleiner als 0,5 mm werden darf, wenn die Viskosität der Emulsion gering genug für eine gute Durchmischung des Fermenters sein soll. Mit den erfindungsgemäß eingesetzten hochhalogenierten Aliphaten wird dieses Ziel erreicht und ein guter Stoffaustausch im Fermentationsmedium gewährleistet.

Ein weiterer Vorteil des erfindungsgemäßen Verfahrens liegt darin, daß die hochhalogenierten Aliphaten nicht mischbar mit hochpolaren, hydrophilen organischen Lösemitteln sind, wie sie bei der Abtrennung von Biopolymeren verwendet werden. Wird also bei der Aufarbeitung das Biopolymere, beispielsweise Xanthan, mit einem niederen Alkohol wie Isopropanol oder einem niederen Keton wie Aceton ausgefällt, so kann aus dem Überstand der hochhalogenierte Aliphat durch einfaches Dekantieren wieder zurückgewonnen werden. Im Gegensatz zu Mineralölen oder fetten Ölen ist also keine destillative Reinigung erforderlich.

Besonders vorteilhaft ist das erfindungsgemäße Verfahren für aerobe Fermentationen, da die hochhalogenierten Aliphaten ein hohes Lösevermögen für Sauerstoff besitzen. Zusätzlich zum besseren Stoffaustausch durch die verringerte Viskosität kommt somit das erhöhte Sauerstoffangebot durch den hochhalogenierten Aliphaten, so daß Umsatz und Ausbeute weiter gesteigert werden können.

Das erfindungsgemäße Verfahren kann auch dann vorteilhaft eingesetzt werden, wenn die Viskosität durch Mycel-Bildung erhöht wird, z.D. bei der Herstellung von Pilz-Metaboliten, und/oder ein verstärktes Rühren zu einer Schädigung des Mikroorganismus und/oder des Produkts führen kann. Weiterhin ist das Verfahren dann vorteilhaft, wenn durch den verbesserten Stoffaustausch eine Produkt-Hemmung der Reaktion verringert oder vermieden wird.

Hochhalogenierte Aliphaten, die unter Fermentationsbedingungen flüssig sind, sind generell wenig toxisch und
finden deshalb als sogenannte Sicherheits-Kühlmittel und
z.T. auch als Blutersatzstoffe Verwendung (Angew. Chem. 90
(1978) 654, I.E. 621). Selbstverständlich ist die Frage
der Giftigkeit für den jeweiligen Mikroorganismus und
die angewandte Dosierung zu entscheiden und kann im Zweifelsfall durch einen Vorversuch ermittelt werden.

Als "hochhalogeniert" im Sinne der Erfindung gelten Aliphaten, bei denen der größte Teil der Wasserstoffatome
durch die Halogene Chlor und insbesondere Fluor ersetzt
ist . Das heißt also, daß auch Verbindungen geeignet sein
können, bei denen noch in untergeordnetem Ausmaß Wasserstoffatome an Kohlenstoff  gebunden sind, weshalb sich
auch technische Gemische mit unterschiedlichem Halogenierungsgrad eignen können.

Bevorzugt sind perhalogenierte Fluor-Chlor-Kohlenwasserstoffe
wie 1,1,2-Trichlor-1,2,2-trifluorethan,  Difluor-tetrachlorethane  oder Trichlor-pentafluor-propane. Geeignet sind
auch verzweigte Verbindungen wie 2,3-Dichlor-2-trifluormethyl-nonafluorpentan (EP-A 37 003). Es ist hierbei nicht erforderlich, daß diese Verbindungen chemisch völlig inert
sind, da bei Fermentationsreaktionen üblicherweise ohnehin
der pH-Wert kontrolliert wird, so daß also eine minimale
Abspaltung von Chlorwasserstoff unschädlich ist.

Besonders bevorzugt sind die hochfluorierten bis perfluorierten Alkane und Alkene mit 8 bis 14 Kohlenstoffatomen, perfluorierte Cycloalkane wie Methyladamantan,
Dimethyladamantan , Decahydronaphthalin und Methyldecahydronaphthalin sowie lineare und cyclische Ether und
Amine, wie sie in Angew.Chem. 90 (1978) 654, I.E. 621 genannt sind.

Die Menge an eingesetztem hochhalogeniertem Aliphaten richtet sich nach den jeweiligen Gegebenheiten und kann durch einfache Vorversuche leicht ermittelt werden. Im allgemeinen werden etwa 10 bis 100, vorzugsweise 40 bis 100 Vol.-%, bezogen auf die wässerige Phase, eingesetzt.

Der hochhalogenierte Aliphat soll bei den Fermentationsbedingungen flüssig sein, d.h. einen Schmelzpunkt von höchstens etwa 25°C und einen Siedepunkt bei Normaldruck von mindestens etwa 40°C aufweisen. Eine geringe Flüchtigkeit erleichtert die Rückgewinnung und verringert die Verluste, insbesondere bei der Aufarbeitung.

In den folgenden Beispielen beziehen sich Prozentangaben auf das Gewicht.

Beispiele:

Eine Kultur von Xanthomonas campestris wird in 6 Ansätze von je 50 ml des folgenden Mediums geimpft: 5 % Glucose, 0,05 % Hefeextrakt, 0,1 % Cornsteep, 0,1 % $NaNO_3$, 0,1 % $KH_2PO_4$, 0,05 % $MgSO_4 \cdot 7H_2O$, 0,003 % $CaCl_2$, 0,001 % Fe(III)-citrat, 0,0002 % $MnSO_4$, 0,0001 % $ZnCl_2$, 0,000025 % $CuSO_4$, 0,000022 % $CoCl_2$, 0,000025 % $Na_2MoO_4 \cdot 2H_2O$ und 0,00001 % Natrium-Ethylendiamintetraacetat.

Zwei Ansätze enthalten zusätzlich je 30 ml, zwei Ansätze zusätzlich je 50 ml Perfluor-1-nonen. Die Kulturen werden 5 Tage bei 30°C in 300 ml-Erlenmeyerkolben geschüttelt (Drehzahl 180 UpM bei 5 cm Schüttelamplitude). Anschließend wird das gebildete Xanthan mit je 60 ml Aceton ausgefällt. Das Perfluor-nonen läßt sich nach Ausfällung des Produktes leicht durch Dekantieren zurückgewinnen.

| Beispiel Nr. | Zusatz (ml) | Ausbeute (%)[*] |
|---|---|---|
| 1 | 30 | 44 |
| 2 | 50 | 48 |
| 3 | – | 36 |

[*] an Polymer, bezogen auf eingesetzten Zucker

**Patentansprüche:**

1. Verfahren zur Verringerung der Viskosität von Fermentationsbrühen durch Zusatz eines halogenierten Kohlenwasserstoffs, dadurch gekennzeichnet, daß der halogenierte Kohlenwasserstoff ein ungiftiger, unter den Fermentationsbedingungen flüssiger, hochhalogenierter Aliphat ist.

2. Verfahren nach Anspruch 1, dadurch gekennzeichnet, daß der hochhalogenierte Aliphat perhalogeniert ist.

3. Verfahren nach Anspruch 1, dadurch gekennzeichnet, daß der hochhalogenierte Aliphat ein hochfluorierter Kohlenwasserstoff, Ether oder ein hochfluoriertes Amin ist.

4. Verfahren nach Anspruch 1 bis 3, dadurch gekennzeichnet, daß man 10 bis 100 %, bezogen auf das Volumen der wässerigen Phase, an hochhalogeniertem Aliphaten zusetzt.

5. Verfahren nach Anspruch 1 bis 4, dadurch gekennzeichnet, daß man 40 bis 100 %, bezogen auf das Volumen der wässerigen Phase, an hochhalogenierten Aliphaten zusetzt.